# EUROPEAN PATENT APPLICATION

(11) **EP 3 449 932 A1**
(43) Date of publication of application: **06.03.2019**
(21) Application number: 17789440.9
(22) Date of filing: 21.04.2017
(51) Int. Cl.: A61K 35/74, A23L 33/135, A61P 1/14, C12N 1/20

(54) **AGENT FOR REGULATING COMPOSITION RATIO OF INTESTINAL BACTERIAL FLORA, DRUG, FOOD, DRINK AND METHOD FOR REGULATING COMPOSITION RATIO OF INTESTINAL BACTERIAL FLORA**

(30) Priority: 26.04.2016 JP 2016088068
(71) Applicant: TFK Co., Ltd, Kobe-shi, Hyogo 652-0884 (JP)
(72) Inventor: TODA Nobuhiro, Kobe-shi Hyogo 652-0884 (JP); HIDAKA Yasuhiro, Kobe-shi Hyogo 652-0884 (JP); SOMA Genichiro, Tokyo 158-0084 (JP); NISHIZAWA Takashi, Takamatsu-shi Kagawa 761-8075 (JP)
(74) Representative: Isarpatent
(86) International application number: PCT/JP2017/016071
(87) International publication number: WO 2017/188157

(57) **Abstract**

The present invention provides a highly-safe agent for regulating the intestinal microbiota distribution ratio. The agent of the present invention contains at least one of a *Rhodobacter azotoformans* BP0899 strain (Accession No. NITE BP-644) or a culture thereof.

SEQUENCE LISTING

<110>  TFK Co.,Ltd.

<120>  Agent for regulating composition ratio of intestinal bacterial flora,
drug, food and method for regulating composition ratio of intestinal
bacterial flora

<130>  TF15092WO

<150>  JP 2016-088068
<151>  2016-04-26

<160>  9

<170>  PatentIn version 3.1

<210>  1
<211>  1427
<212>  DNA
<213>  Rhodobacter azotoformans

<400>  1
gagtttgatc ctggctcaga atgaacgctg gcggcaggcc taacacatgc aagtcgagcg      60

aagtcttcgg acttagcggc ggacgggtga gtaacgcgtg ggaacatgcc caaaggtacg     120

gaatagcccc gggaaactgg gagtaatacc gtatgtgccc ttcgggggaa agatttatcg     180

cctttggatt ggcccgcgtt ggattaggta gttggtgggg taatggccta ccaagccgac     240

gatccatagc tggtttgaga ggatgatcag ccacactggg actgagacac ggcccagact     300

cctacgggag gcagcagtgg ggaatcttag acaatgggcg caagcctgat ctagccatgc     360

cgcgtgatcg atgaaggcct tagggttgta aagatctttc aggtgggaag ataatgacgg     420

taccaccaga agaagccccg gctaactccg tgccagcagc cgcggtaata cggagggggc     480

tagcgttatt cggaattact gggcgtaaag cgcacgtagg cggactggaa agtcaggggt     540

gaaatcccgg ggctcaaccc cggaactgcc tttgaaactc ccagtcttga ggtcgagaga     600

ggtgagtgga attccgagtg tagaggtgaa attcgtagat attcggagga acaccagtgg     660

cgaaggcggc tcactggctc gatactgacg ctgaggtgcg aaagcgtggg gagcaaacag     720

gattagatac cctggtagtc cacgccgtaa acgatgaatg ccagtcgtcg ggcagcatgc     780

tgttcggtga cacacctaac ggattaagca ttccgcctgg ggagtacggc cgcaaggtta     840

aaactcaaag gaattgacgg gggcccgcac aagcggtgga gcatgtggtt taattcgaag     900

caacgcgcag aaccttacca acccttgaca tggcgatcgc ggttccagag atggttcctt     960

cagttcggct ggatcgcaca caggtgctgc atggctgtcg tcagctcgtg tcgtgagatg    1020

ttcggttaag tccggcaacg agcgcaaccc acgtcctcag ttgccagcat tcagttgggc    1080

actctgggga aactgccggt gataagccgg aggaaggtgt ggatgacgtc aagtcctcat    1140

ggcccttacg ggttgggcta cacacgtgct acaatggcag tgacaatggg ttaatcccaa    1200

aaagctgtct cagttcggat tggggtctgc aactcgaccc catgaagtcg gaatcgctag   1260

taatcgcgta acagcatgac gcggtgaata cgttcccggg ccttgtacac accgcccgtc   1320

acaccatggg aattggttct acccgaaggc ggtgcgccaa cctcgcaaga ggaggcagcc   1380

gaccacggta ggatcagtga ctggggtgaa gtcgtaacaa ggtagcc                 1427


<210>  2
<211>  19
<212>  DNA
<213>  Artificial

<220>
<223>  a primer

<400>  2
gagtttgatc ctggctcag                                                  19


<210>  3
<211>  19
<212>  DNA
<213>  Artificial

<220>
<223>  a primer

<400>  3
ctcctacggg aggcagcag                                                  19


<210>  4
<211>  21
<212>  DNA
<213>  Artificial

<220>
<223>  a primer

<400>  4
ggattagata ccctggtagt c                                               21


<210>  5
<211>  16
<212>  DNA
<213>  Artificial

<220>
<223>  a primer

<400>  5
gcaacgagcg caaccc                                                     16


<210>  6
<211>  18
<212>  DNA
<213>  Artificial

<220>

<223>  a primer

<400>  6
gtattaccgc ggctgctg                                                    18


<210>  7
<211>  18
<212>  DNA
<213>  Artificial

<220>
<223>  a primer

<400>  7
taccagggta tctaatcc                                                    18


<210>  8
<211>  16
<212>  DNA
<213>  Artificial

<220>
<223>  a primer

<400>  8
ccattgtagc acgtgt                                                      16


<210>  9
<211>  17
<212>  DNA
<213>  Artificial

<220>
<223>  a primer

<400>  9
aaggaggtga tccagcc                                                     17

## Description

### TECHNICAL FIELD

The present invention relates to an agent for regulating the intestinal microbiota (an intestinal bacterial flora) distribution ratio, a pharmaceutical, food, drink, and a method for regulating the intestinal microbiota distribution ratio.

### BACKGROUND ART

It has been recently reported that obesity can be improved by regulating the intestinal microbiota distribution ratio. For example, the patent literature 1 proposes a weight increase inhibitor, a neutral fat-reducing agent, and a slimming agent, which regulate the intestinal microbiota distribution ratio by extracts of plants belonging to genus Salacia (patent literature 1).

### Citation List

### Patent Literature

Patent Literature 1: JP 2015-127340 A

### SUMMARY OF INVENTION

### Technical Problem

However, a novel, highly-safe technique for regulating the intestinal microbiota distribution ratio using a novel material has been required.

Hence, the present invention is intended to provide a novel, highly-safe agent and method for regulating the intestinal microbiota distribution ratio.

### Solution to Problem

In order to achieve the aforementioned object, the present invention provides an agent for regulating the intestinal microbiota distribution ratio, including at least one of a *Rhodobacter azotoformans* BP0899 strain (Accession No. NITE BP-644) or a culture thereof.

The present invention further provides a method for regulating the intestinal microbiota distribution ratio, including the step of administering an agent for regulating the intestinal microbiota distribution ratio, the agent including at least one of a *Rhodobacter azotoformans* BP0899 strain (Accession No. NITE BP-644) or a culture thereof.

### Advantageous Effects of Invention

In order to achieve the aforementioned object, the inventors of the present invention conducted a series of studies and found that the intestinal microbiota distribution ratio can be regulated by administering at least one of *Rhodobacter azotoformans* (Accession No. NITE BP-644) BP0899 strain or a culture thereof and reached the present invention. At least one of the *Rhodobacter azotoformans* BP0899 strain (Accession No. NITE BP-644) or a culture thereof is highly safe and can be administered for a long period of time.

### BRIEF DESCRIPTION OF DRAWINGS

[FIG. 1] FIG. 1 is a graph showing a change in distribution ratio of genus *Bacteroides* in the intestinal microbiota of mice in the example.
[FIG. 2] FIG. 2 is a graph showing a change in distribution ratio of genus *Lactobacillus* in the intestinal microbiota of mice in the example.
[FIG. 3] FIG. 3 is a graph showing a change in distribution ratio of genus *Prevotella* in the intestinal microbiota of mice in the example.
[FIG. 4] FIG. 4 is a graph showing a change in distribution ratio of *Clostridium* claster XVIII in the intestinal microbiota of mice in the example.
[FIG. 5] FIG. 5 is a graph showing a change in distribution ratio of *Clostridium* subclaster XIVa in the intestinal microbiota of mice in the example.
[FIG. 6] FIG. 6 is a graph showing a change in distribution ratio of *Clostridium* claster XI in the intestinal microbiota of mice in the example.
[FIG. 7] FIG. 7 is a graph showing a change in distribution ratio of genus *Bacteroides* in the intestinal microbiota of mice in the example.
[FIG. 8] FIG. 8 is a graph showing a change in distribution ratio of genus *Lactobacillus* in the intestinal microbiota of mice in the example.
[FIG. 9] FIG. 9 is a graph showing a change in distribution ratio of genus *Prevotella* in the intestinal microbiota of mice in the example.
[FIG. 10] FIG. 10 is a graph showing a change in distribution ratio of *Clostridium* claster XVIII in the intestinal microbiota of mice in the example.
[FIG. 11] FIG. 11 is a graph showing a change in distribution ratio of *Clostridium* subclaster XIVa in the intestinal microbiota of mice in the example.
[FIG. 12] FIG. 12 is a graph showing a change in distribution ratio of *Clostridium* claster XI in the intestinal microbiota of mice in the example.

### DESCRIPTION OF EMBODIMENTS

In the agent for regulating the intestinal microbiota distribution ratio of the present invention, at least one of a *Rhodobacter azotoformans* BP0899 strain (Accession No. NITE BP-644) or a culture thereof preferably has the following mycological characteristics (1) to (30). The BP0899 strain was deposited with International Patent Organism Depositary, the National Institute of Technology and Evaluation (2-5-8, Kazusakamatari, Kisarazu-shi, Chiba, Japan) under the Accession No. NITE P-644 (deposition date: September 12, 2008) and was internationally deposited under the Accession No. NITE BP-644 (transfer date: October 27, 2010).
(1) cell morphology: a rod shape or an oval shape;
(2) polymorphism: none;
(3) cell size: 0.8 µm × 1.0 µm;
(4) the presence or absence of motility: present;
(5) the presence or absence of spore: absent;
(6) luster in nutrient agar culture: positive;
(7) pigment production in nutrient agar culture: positive;
(8) the presence or absence of surface growth in nutrient broth culture: absent;
(9) the presence or absence of medium turbidity in nutrient broth culture: present;
(10) liquefaction of gelatin in gelatin stab culture: negative;
(11) coagulation in litmus-milk culture: negative;
(12) liquefaction in litmus-milk culture: negative;
(13) Gram staining properties: negative;
(14) reduction of nitrate: negative;
(15) denitrification: positive/negative;
(16) MR test: negative;
(17) production of indole: negative;
(18) production of hydrogen sulfide: negative;
(19) hydrolysis of starch: negative;
(20) utilization of citric acid (Christensen): negative;
(21) utilization of inorganic nitrogen source (ammonium salt): positive;
(22) production of catalase: positive;
(23) production of oxidase: positive;
(24) anaerobic growth: positive;
(25) O-F test (oxidation/fermentation): negative/negative;
(26) β-galactosidase activity: negative;
(27) arginine dihydrolase activity: negative;
(28) lysine decarboxylase activity: negative;
(29) tryptophan deaminase activity: negative; and
(30) gelatinase activity: negative.

In the agent for regulating the intestinal microbiota distribution ratio of the present invention, a base sequence of 16S-rRNA in the BP0899 strain is preferably represented by SEQ ID NO: 1.

The agent for regulating the intestinal microbiota distribution ratio of the present invention preferably increases a distribution ratio or inhibits a reduction in the distribution ratio of at least one intestinal bacteria selected from the group consisting of genus *Bacteroides,* genus *Lactobacillus,* genus *Prevotella*, *Clostridium* claster XVIII, *Clostridium* subclaster XIVa, and *Clostridium* claster XI.

The present invention further provides a pharmaceutical for regulating the intestinal microbiota distribution ratio, including the agent for regulating the intestinal microbiota distribution ratio of the present invention.

The present invention further provides food and drink with the intestinal microbiota distribution ratio regulatory function, including the agent for regulating the intestinal microbiota distribution ratio of the present invention.

The following describes the present invention in detail.

As mentioned above, the present invention provides an agent for regulating the intestinal microbiota distribution ratio, including at least one of a *Rhodobacter azotoformans* BP0899 strain (Accession No. NITE BP-644) or a culture thereof. The agent for regulating the intestinal microbiota distribution ratio of the present invention may contain at least one of purple non-sulfur bacteria or a culture thereof. The purple non-sulfur bacteria may include *Rhodobacter azotoforman*, and the *Rhodobacter azotoformans* may include a *Rhodobacter azotoformans* BP0899 strain (Accession No. NITE BP-644).

### (Bacteria)

In the present invention, at least one of a BP0899 strain, which is purple non-sulfur bacteria, or a culture thereof has, as mentioned above, the following mycological characteristics (1) to (30):
(1) cell morphology: a rod shape or an oval shape;
(2) polymorphism: none;
(3) cell size: 0.8 µm × 1.0 µm;
(4) the presence or absence of motility: present;
(5) the presence or absence of spore: absent;
(6) luster in nutrient agar culture: positive;
(7) pigment production in nutrient agar culture: positive;
(8) the presence or absence of surface growth in nutrient broth culture: absent;
(9) the presence or absence of medium turbidity in nutrient broth culture: present;
(10) liquefaction of gelatin in gelatin stab culture: negative;
(11) coagulation in litmus-milk culture: negative;
(12) liquefaction in litmus-milk culture: negative;
(13) Gram staining properties: negative;
(14) reduction of nitrate: negative;
(15) denitrification: positive/negative;
(16) MR test: negative;
(17) production of indole: negative;
(18) production of hydrogen sulfide: negative;
(19) hydrolysis of starch: negative;
(20) utilization of citric acid (Christensen): negative;
(21) utilization of inorganic nitrogen source (ammonium salt): positive;
(22) production of catalase: positive;
(23) production of oxidase: positive;
(24) anaerobic growth: positive;
(25) O-F test (oxidation/fermentation): negative/negative;
(26) β-galactosidase activity: negative;
(27) arginine dihydrolase activity: negative;
(28) lysine decarboxylase activity: negative;
(29) tryptophan deaminase activity: negative; and
(30) gelatinase activity: negative.

At least one of a BP0899 strain, which is purple non-sulfur bacteria, or a culture thereof may have properties shown in the following mycological characteristic (31) under aerobic culturing conditions in the dark. In the following mycological characteristic (31), "-" refers to the absence of production, and "+" refers to the presence of production.

(31) Acid production and gas production from saccharide

| Substrate | Acid production/Gas production |
|---|---|
| L-arabinose | -/- |
| D-glucose | -/- |
| D-fructose | -/- |
| maltose | -/- |
| lactose | -/- |
| D-sorbitol | -/- |
| inositol | -/- |
| D-xylose | -/- |
| D-mannose | -/- |
| D-galactose | -/- |
| saccharose | -/- |
| trehalose | -/- |
| glycerin | -/- |

The mycological characteristics may be evaluated from the results obtained after main culturing conducted after preculturing. The preculturing may be conducted by inoculating the BP0899 strain, which is purple non-sulfur bacteria, onto a nutrient agar medium, which is then cultured for 24 hours at 30°C. The conditions of the main culturing can be set, as appropriate, according to the method for evaluating each of the mycological characteristics. Specifically, culturing conditions for the mycological characteristics (1) to (5) are, for example, aerobic culturing on a nutrient agar medium at 30°C in the dark. The culturing conditions for the mycological characteristics (6) and (7) are, for example, anaerobic culturing in a nutrient broth medium at 30°C in the light. The culturing conditions for the mycological characteristics (8) to (12) are, for example, aerobic culturing in each medium at 30°C in the dark. The culturing conditions for the mycological characteristics (13), (14), (16), (17), and (19) to (23), an oxidation test of the mycological characteristic (25), and the mycological characteristics (26), (29), (30), and (31) are, for example, aerobic culturing in the dark. The culturing conditions for the mycological characteristics (15), (18), (24), a fermentation test of the mycological characteristic (25), and the mycological characteristics (27) and (28) are, for example, anaerobic culturing in the dark. A method for testing each of these mycological characteristics is not limited to particular methods, and a conventionally-known method can be employed. Specific examples thereof include methods described in publications: Barrow G.I. and Feltham R.K.A.: "Cowan and Steel's Manual for the Identification of Medical Bacteria.", 3rd edition, Cambridge University Press, England (1993); SAKAZAKI et al.: "Shin Saikin-baichi-gaku Koza, PART II (The Course of Culturing Medium for Microorganism, PART II)", 2nd edition, Kindai Shuppan, Tokyo (1988); Hasegawa: "Biseibutu no Bunrui to Dotei, PART II (Classification and Determination of Microorganism, PART II)", Gakkai Shuppan Center, Tokyo (1985); and Japanese Society of Soil Micobiology: "Shinpen Dojou-biseibutsu-gaku Jikken (Soil Micoorganism Experimental Method, New Edition)", Yokando, Tokyo (1992). As a method for testing (15), a method of KOMAGATA et al., described in "Biseibutu no Bunrui to Dotei, PART II", a method using a Giltay medium, described in "Shinpen Dojou-biseibutsu-gaku Jikken", or a sewage water testing method using a PYN medium can be employed, for example. In the method of KOMAGATA et al., when growth of the purple non-sulfur bacteria and gas formation are recognized in broth containing 1 % sodium nitrate under anaerobic culturing conditions, it is determined that denitrification is positive. In the method using a Giltay medium, when gas is generated in a Giltay medium (pH 7.0 to 7.2) containing a Durham tube, and the Giltay medium turns deep blue in color, under anaerobic culturing conditions, it is determined that denitrification is positive. The Giltay medium is a medium obtained by mixing a liquid A (containing 1 g of KNO₃, 1 g of asparagine, 5 ml of 1 % bromthymol blue-alcohol solution, and 500 ml of distilled water) and a liquid B (containing 8.5 g of sodium citrate, 1 g of MgSO₄·7H₂O, 0.05 g of FeCl₃·6H₂O, 1 g of KH₂PO₄, 0.2 g of CaCl₂·6H₂O, and 500 ml of distilled water). In the method for testing each of the mycological characteristics, a commercially available bacterial identification kit may be used, for example. The kit is not limited to particular kits, and for example, a bacterial identification kit API20E (produced by SYSMEX bioMerieux Co., Ltd.) can be used.

At least one of a BP0899 strain, which is the purple non-sulfur bacteria, or a culture thereof may further have the following mycological characteristics (32) to (40):
(32) color of colony: red;
(33) gelatin stab culture: no growth;
(34) VP test: negative;
(35) utilization of citric acid (Koser): positive;
(36) utilization of inorganic nitrogen source (nitrate salt): positive;
(37) urease activity: negative;
(38) growth pH range: 5 to 9;
(39) acid production from D-mannitol: positive; and
(40) gas production from D-mannitol: negative.

A method for testing each of these mycological characteristics (32) to (40) is not limited to particular methods, and a conventionally-known method can be employed. Specific examples thereof include the methods described in the above-mentioned publications. In the method, a commercially available bacterial identification kit may be used, for example. The kit is not limited to particular kits, and the above-mentioned bacterial identification kit can be used, for example.

The agent for regulating the intestinal microbiota distribution ratio of the present invention may further contain, for example, other purple non-sulfur bacteria besides at least one of a BP0899 strain or a culture thereof.

The other purple non-sulfur bacteria are not limited to particular bacteria, and examples thereof include bacteria of genus *Rhodospirillum*, genus *Rhodocista*, genus *Rhodopila*, genus *Rhodomicrobium*, genus *Blastochloris*, genus *Rhodoplanes*, genus *Rhodobium*, genus *Rhodocyclus*, genus *Rhodoferax*, and genus *Rhodopseudomonas.*

The other purple non-sulfur bacteria may be, for example, *Rhodobacter azotoformans* other than at least one of a *Rhodobacter azotoformans* BP0899 strain (Accession No. NITE BP-644) or a culture thereof and bacteria belonging to genus *Rhodobacter* other than the *Rhodobacter azotoformans.*

A source from which the purple non-sulfur bacteria including a BP0899 strain (hereinafter referred to as "purple non-sulfur bacteria") is collected is not limited to particular sources and examples thereof include the ground, seawater, river water, lake water, and boggy water. The ground is not particularly limited, and examples thereof include a soil, sands, mud, and the like on land, the sea bottom, a river bottom, a lake bottom, and a bog bottom.

A method for isolating the purple non-sulfur bacteria is not limited to particular methods, and for example, a conventionally-known collection method, a conventionally-known culturing method, or the like can be used. When the source is lake water, the method may include filtration of collected lake water with a filter or the like, culturing of a filtrate thus obtained on an agar medium or the like, and isolation of the purple non-sulfur bacteria from a colony thus obtained. When the source is mud, the method may include suspension of collected mud in a buffer solution or the like, centrifugation of this suspended solution thus obtained, culturing of a supernatant thus obtained on an agar medium or the like, and isolation of the purple non-sulfur bacteria from a colony thus obtained. The purple non-sulfur bacteria thus isolated may be cultured in a liquid medium.

The agent for regulating the intestinal microbiota distribution ratio of the present invention may further contain other bacteria besides the purple non-sulfur bacteria. The other bacteria are not limited to particular bacteria, and examples thereof include lactobacillus and yeast. The other bacteria are preferably lactobacillus.

The lactobacillus is not particularly limited, and examples thereof include *Lactobacillus acidphilus*, *Lactobacillus casei*, *Lactobacillus lactis, Lactobacillus bulgaricus, Lactobacillus helveticus*, *Lactobacillus delbrueckii, Lactobacillus plantarum*, *Lactobacillus brevis, Lactococcus lactis, Bifidobacterium longum, Bifidobacterium breve, Enterococcus faecalis, Streptococcus thermophilus*, and *Streptococcus lactis*, and preferred examples thereof include *Lactobacillus acidphilus*, *Lactobacillus bulgaricus*, *Streptococcus thermophilus*, and *Streptococcus lactis.*

The yeast is not particularly limited, and examples thereof include *Saccharomyces cerevisiae*, *Saccharomyces carlsbergensis*, *Saccharomyces ellipsoideus*, and *Saccharomyces rouxii.*

A medium used in culturing of a purple non-sulfur bacteria is not limited to particular media, and examples thereof include a low-chain fatty acid-containing medium, a maleic acid-containing medium, a medium for reactivating L-dried cultures of bacteria 802, "Daigo" (manufactured by NIHON PHARMACEUTICAL CO., LTD.), a MYS medium (HIRAISHI and KITAGAWA: Bulletin of the Japanese Society of Scientific Fisheries, vol. 50(11), pp.1929 to 1937 (1984)), a modified MYS medium, and a growth medium. Preferably, the medium is the low-chain fatty acid-containing medium, the maleic acid-containing medium, or the medium for reactivating L-dried cultures of bacteria 802, "Daigo" (manufactured by NIHON PHARMACEUTICAL CO., LTD.).

The low-chain fatty acid-containing medium and the maleic acid-containing medium each can be, for example, a medium obtained by containing biotin, Vitamin B₁, nicotinic acid, low-chain fatty acid, or a maleic acid sodium salt in a basal medium shown in Table 1 below. The low-chain fatty acid is not limited to particular acids, and preferred examples thereof include acetic acid, propionic acid, and lactic acid.

**[Table 1]**

| (Basal medium) | |
|---|---|
| Component | Component concentration (w/v%) |
| (NH₄)₂SO₄ | 0.03 |
| KH₂PO₄ | 0.05 |
| MgSO₄·7H₂O | 0.02 |
| NaCl | 0.05 |
| NaHCO₃ | 0.02 |
| Yeast extract | 0.001 |

The modified MYS medium can be, for example, a medium having a composition shown in Table 2 below. The growth medium can be, for example, a medium having a composition shown in Table 3 below.

**[Table 2]**

| (Composition of modified MYS medium) | |
|---|---|
| Component | Concentration |
| Sodium malate | 3.6 g/l |
| Yeast extract | 0.5 g/l |
| (NH₄)₂SO₄ | 1.0 g/l |
| KH₂PO₄ | 1.0 g/l |
| MgCl₂·6H₂O | 0.2 g/l |
| NaCl | 0.2 g/l |
| CaCl₂·2H₂O | 0.045 g/l |
| EDTA-2Na | 2 mg/l |
| FeSO₄·7H₂O | 2 mg/l |
| H₃BO₃ | 0.1 mg/l |
| CoCl₂·6H₂O | 0.1 mg/l |
| ZnCl₂ | 0.1 mg/l |
| MnCl₂·4H₂O | 0.1 mg/l |
| Na₂MoO₄·2H₂O | 0.02 mg/l |
| NiCl₂·6H₂O | 0.02 mg/l |
| CuCl₂·2H₂O | 0.01 mg/l |
| Na₂SeO₃ | 0.001 mg/l |
| Vitamin B₁-HCl | 0.5 mg/l |
| Nicotinic acid | 0.5 mg/l |
| p-aminobenzoate | 0.3 mg/l |
| Vitamin B₁₂ | 0.05 mg/l |
| Vitamin B₆-HCl | 0.1 mg/l |
| Vitamin H | 0.05 mg/l |

**[Table 3]**

| (Composition of growth medium) | |
|---|---|
| Component | Concentration |
| Sodium acetate | 3 g/l |
| Sodium lactate | 3 g/l |
| Sodium butyrate | 3 g/l |
| Sodium chloride | 5 g/l |
| L-glutamic acid | 0.17 g/l |
| K₂HPO₄ | 1 g/l |
| KH₂PO₄ | 1.5 g/l |
| EDTA | 20 mg/l |
| CaCl₂ | 70 mg/l |
| H₃BO₃ | 3 mg/l |
| CoCl₂·6H₂O | 0.95 mg/l |
| ZnSO₄·7H₂O | 0.24 mg/l |
| Cu(NO₃)₂·3H₂O | 0.04 mg/l |
| NiCl₂·6H₂O | 0.02 mg/l |
| MgSO₄·7H₂O | 0.2 g/l |
| MnSO₄·5H₂O | 2 mg/l |
| Na₂MoO₄·2H₂O | 1 mg/l |
| FeSO₄·7H₂O | 20 mg/l |
| Biotin | 0.05 mg/l |
| VitaminB₁-HCl | 5 mg/l |
| Nicotinic acid | 5 mg/l |
| Yeast extract | 0.02 g/l |

The temperature range in the culturing is not limited to particular temperature ranges, and is, for example, the range from 23°C to 39°C, and preferably 30°C.

The pH range in the culturing is also not limited to particular pH ranges, and is, for example, the range from 5.5 to 8.5, preferably from 6.0 to 8.5, and more preferably 7.0.

The culturing may be conducted under aerobic conditions or anaerobic conditions and is not particularly limited. However, it is preferred that the culturing is conducted under anaerobic conditions. Light conditions in the culturing are also not limited to particular conditions and may be darkness conditions or illuminated conditions. However, it is preferred that the light conditions are conditions under which illuminance is from 2000 to 10000 lux. The culturing may be conducted in a sealed and illuminated culture vessel. The culturing may also be conducted while stirring a culture solution using a stirrer provided in the sealed and illuminated culture vessel.

The culturing time is not particularly limited, and may be, for example, the time until growth of the purple non-sulfur bacteria reaches the stationary phase. Under the culturing conditions under which growth of the purple non-sulfur bacteria reaches the stationary phase in about 72 hours, the culturing time may be, for example, 72 hours.

In the culturing of the purple non-sulfur bacteria, only the purple non-sulfur bacteria may be cultured, or a mixed culturing of the purple non-sulfur bacteria and other bacteria may be conducted, for example. The other bacteria are not limited to particular bacteria, and examples thereof include the above-mentioned lactobacillus and the above-mentioned yeast.

As mentioned above, a base sequence of 16S-rRNA in the BP0899 strain is preferably represented by SEQ ID NO: 1.

The base sequence of 16S-rRNA can be identified using a primer and the like after isolating and culturing the BP0899 strain and extracting DNA therefrom by the above-mentioned method. As a method for extracting DNA and a method for identifying a base sequence, common methods can be used, and the method is not limited to particular methods. The primer is not particularly limited, and for example, the following primers can be used.

### (Primer)

9F (SEQ ID NO: 2)
   5'-GAGTTTGA TCCTGGCTCAG-3'
339F (SEQ ID NO: 3)
   5'-CTCCTACGGGAGGCAGCAG-3'
785F (SEQ ID NO: 4)
   5'-GGATTAGATACCCTGGTAGTC-3'
1099F (SEQ ID NO: 5)
   5'-GCAACGAGCGCAACCC-3'
536R (SEQ ID NO: 6)
   5'-GTATTACCGCGGCTGCTG-3'
802R (SEQ ID NO: 7)
   5'-TACCAGGGTATCTAATCC-3'
1242R (SEQ ID NO: 8)
   5'-CCATTGTAGCACGTGT-3'
1541R (SEQ ID NO: 9)
   5'-AAGGAGGTGATCCAGCC-3'

### (Culture)

Examples of a culture obtained by culturing the purple non-sulfur bacteria include bacterial cells of the purple non-sulfur bacteria, a culture supernatant of the purple non-sulfur bacteria, and an extract from the bacterial cells of the purple non-sulfur bacteria, and the culture is not limited to particular cultures. The agent for regulating the intestinal microbiota distribution ratio of the present invention may further contain a culture obtained by culturing bacteria other than the purple non-sulfur bacteria. The culture obtained by culturing bacteria other than the purple non-sulfur bacteria is not limited to particular cultures, and examples thereof include bacterial cells of the other bacteria, a culture supernatant of the other bacteria, and an extract from the bacterial cells of the other bacteria. Specific examples of the culture obtained by culturing bacteria other than the purple non-sulfur bacteria include dried bacterial cells of the lactobacillus and the yeast and extracts therefrom.

Examples of the culture include a treated material of the bacterial cells, a treated material of the culture supernatant, and a treated material of the extract of the bacterial cells, and the culture is limited to particular cultures. The treated materials are not limited to particular materials, and examples thereof include a substance obtained by concentrating the culture, a substance obtained by drying the the culture, a substance obtained by freeze-drying the culture, a substance obtained by treating the culture with a solvent, a substance obtained by treating the culture with a surfactant, a substance obtained by treating the culture with an enzyme, a substance obtained by fractionating a protein of the culture, a substance obtained by sonicating the culture, and a substance obtained by disintegrating the culture. The culture may be, for example, a mixture of the bacterial cells, the culture supernatant, the extract from the bacterial cells, the treated material of the bacterial cells, the treated material of the culture supernatant, and the treated material of the extract of the bacterial cells. The mixture can be obtained by mixing them in any combination and any ratio and is not limited to particular mixtures. The combination is not limited to particular combinations and can be, for example, a mixture of the bacterial cells and the culture supernatant.

The agent for regulating the intestinal microbiota distribution ratio of the present invention may further contain other components such as additives, for example. The additives are not limited to particular additives and can be, for example, a stabilizing agent and the like. A method for producing the agent for regulating the intestinal microbiota distribution ratio is not limited to particular methods and a usually-used formulation technology or the like can be employed.

As demonstrated in the examples below, the agent for regulating the intestinal microbiota distribution ratio of the present invention can increase a distribution ratio or can inhibit the reduction in the distribution ratio of intestinal bacteria such as, for example, genus *Bacteroides,* genus *Lactobacillus,* genus *Prevotella*, *Clostridium* claster XVIII, *Clostridium* subclaster XIVa, and *Clostridium* claster XI.

Fukuda et al. has reported as follows. Short-chain fatty acids such as butyric acid generated from some of intestinal bacteria activate a peroxisome proliferator-activated receptor γ of adipocyte, resulting in reduction of incidence rate of diabetes and promotion of insulin secretion and appetite-suppressing action. As specific intestinal bacteria, *Clostridium* subclaster XIVa is disclosed (Fukuda et al. (2014), Experimental Medicine, 32(5), p.726-732, "function of the intestinal microbiota, elucidated by transomics"). Moreover, according to "Feature: Resent advances in PPARγ agonist research", Japanese journal of clinical medicine (Nipponrinsho), 2010, volume 68, No. 2, p. 176-360, the activation of peroxisome proliferator-activated receptor γ causes improvement in anti-vascular failures, cardiovascular diseases such as dyslipidemia and arteriosclerosis, gastrointestinal diseases, renal diseases, malignant tumors, and the Alzheimer's disease and has immunomodulation action. The agent for regulating the intestinal microbiota distribution ratio of the present invention can increase, for example, the distribution ratio of such intestinal bacteria that generate shirt-chain fatty acids such as butyric acid. Specifically, by administering the agent for regulating the intestinal microbiota distribution ratio of the present invention, the distribution ratio of *Clostridium* subclaster XIVa that generates short-chain fatty acids such as butyric acid can be increased, for example. The agent for regulating the intestinal microbiota distribution ratio of the present invention thus can improve, for example, diabetes, obesity, cardiovascular disease such as dyslipidemia and arteriosclerosis, gastrointestinal disease, renal disease, malignant tumors, and the Alzheimer's disease and has immunomodulation action. Moreover, the patent literature 1 has reported that the distribution ratio of intestinal genus *Bacteroides* is reduced in human subjects with obesity. Thus, for example, it is considered that obesity can be improved by increasing the distribution ratio of genus *Bacteroides* through administering the agent for regulating the intestinal microbiota distribution ratio of the present invention. Furthermore, Miyake et al. has reported that the distribution ratio of intestinal genus *Prevotella* and the distribution ratio of intestinal *Clostridium* subclaster XIVa are reduced in subjects with multiple sclerosis (Miyake S. et al., (2015), PLOS One, 10.e0137429). Thus, for example, it is considered that multiple sclerosis can be improved by increasing the distribution ratio of at least one of intestinal genus *Prevotella* or intestinal *Clostridium* subclaster XIVa through administering the agent for regulating the intestinal microbiota distribution ratio of the present invention. Moreover, Filip et al. has reported that the distribution ratio of intestinal genus *Prevotella* is reduced in subjects with Parkinson's disease (Filip S. et al., (2015), Movement Disorders, Vol. 30, No. 3, p. 350-358). Thus, for example, it is considered that Parkinson's disease can be improved by increasing the distribution ratio of genus *Prevotella* through administering the agent for regulating the intestinal microbiota distribution ratio of the present invention. Furthermore, the genus *Lactobacillus* is also widely known as probiotics. Therefore, for example, it is considered that constipation can be improved by increasing the distribution ratio of genus *Lactobacillus* through administering the agent for regulating the intestinal microbiota distribution ratio of the present invention.

### (Pharmaceutical)

The pharmaceutical of the present invention is for regulating the intestinal microbiota distribution ratio and is not at all limited except that it contains the agent for regulating the intestinal microbiota distribution ratio of the present invention. In the present invention, the pharmaceutical includes pharmaceuticals and quasi drugs.

Examples of a dosage form of the pharmaceutical include a powder, a fine granule, a granule, a tablet, a coated tablet, a capsule, a troche, and a liquid, and the dosage form is not limited to particular forms. Composition of the pharmaceutical is not limited to a particular composition, and the pharmaceutical may contain various additives such as a diluent, a binder, a lubricant, a disintegrant, sorbefacient, an emulsifier, a stabilizing agent, and a preservative. The pharmaceutical can be produced using a usually-used formulation technology or the like. An animal species into which the pharmaceutical is administered is not limited to particular species, and examples thereof include: mammals such as human and non-human such as monkeys, cattle, pigs, dogs, and cats; bird species such as chickens; and fish and seafood. A method for administering the pharmaceutical is not limited to particular methods, and examples thereof include oral administration and parenteral administration. Examples of the parenteral administration include percutaneous absorption, an injection, and administration of a suppository. An administration amount of the pharmaceutical can be set, as appropriate, according to the animal species, ages, and the like. In the method for regulating the intestinal microbiota distribution ratio of the present invention, an administration method and an administration target are the same as those of the pharmaceutical of the present invention, for example.

### (Food and drink)

The food and drink of the present invention has the intestinal microbiota distribution ratio regulation function and is not at all limited except that it contains the agent for regulating the intestinal microbiota distribution ratio of the present invention. In the present invention, food and drink encompasses common food and food with health claims. The common food is not limited to particular foods, and examples thereof include processed grain products, processed vegetable products, processed fruit products, processed meat products, processed fishery products, dairy products, beverages, and health food. The food and drink of the present invention may contain the agent for regulating the intestinal microbiota distribution ratio as a material or an additive, for example. The processed grain products are not limited to particular products, and examples thereof include flour, rice flour, cereal bars, senbei (rice crackers), arare (rice cake cubes), and cookies. The processed vegetable products are not limited to particular products, and examples thereof include vegetable pastes, dried vegetables, and vegetable soups. The processed fruit products are not limited to particular products, and examples thereof include pureed fruits and dried fruits. The processed meat products are not limited to particular products, and examples thereof include hams, bacon, and sausages. The processed fishery products are not limited to particular products, and examples thereof include tsukudani (foods boiled down in soy sauce), enkanbutsu (salted-dried foods), fish sausages, hanpen (boiled fish paste cake), kamaboko (steamed fish paste cake), and chikuwa (tube-shaped fish paste cake). The dairy products are not limited to particular products, and examples thereof include milk beverages, yoghurts, ice creams, and cheeses. The beverages are not limited to particular beverages, and examples thereof include soft drinks, green tea, black tea, and coffee. The food with health claims is generally also called functional food. Examples of the food with health claims include food for specified health use, food with nutrient function claims, and food with nutrient function claims.

The composition of the food and drink is not limited to particular composition and can include, for example, various food materials, auxiliary agents, and stabilizing agents besides the agent for regulating the intestinal microbiota distribution ratio. The food and drink can be produced using a usually-used formulation technology or the like. An animal species by which the food and drink is taken is not limited to particular species, and examples thereof include: mammals such as human and inhuman such as monkeys, cattle, pigs, dogs, and cats; bird species such as chickens; and fish and seafood.

### Examples

The following describes examples of the present invention. The present invention, however, is by no means limited thereto.

### (1) Administration of BP0899 strain suspension to mice

Male C57BL/6J mice at 5 weeks of age were fed with a normal diet (CE-2 solid sample, manufactured by CLEA Japan, Inc.) and water for two days and then fed with a normal diet (AIN-93M purified sample, manufactured by American Institute of Nutrition) for five days. Subsequently, distilled water was added to 100 mg of the BP0899 strain to make the mixture up to 10 mL, and the mixture was stirred with a Vortex mixer to prepare a suspension. Thereafter, 9 mL of distilled water was added to 1 mL of the suspension to prepare 10 mL of the mixture, and this mixture was used as a BP0899 strain suspension. The mice at 6 weeks of age were divided into a group (Example 1-1, 7 mice) where the intake of the BP0899 strain is 10 mg/kg and a group (Example 1-2, 7 mice) where the intake of the BP0899 strain is 100 mg/kg. The day after the division was the start day of administration (day 1), and the BP0899 strain suspension was orally administered to each group with sonde from day 1 to day 14. Moreover, a control group for which the same experiments as in Examples 1-1 and 1-2 were conducted except that distilled water was administered as a substitute for the BP0899 strain suspension was used as a comparative example (Comparative Example 1, 7 mice). All of three groups of Examples 1-1 and 1-2 and Comparative Example 1 were subjected to analysis of feces shown below.

### (2) Analysis 1 of feces (Comparison between distribution ratios of intestinal bacteria (%) on day 3 and day 7)

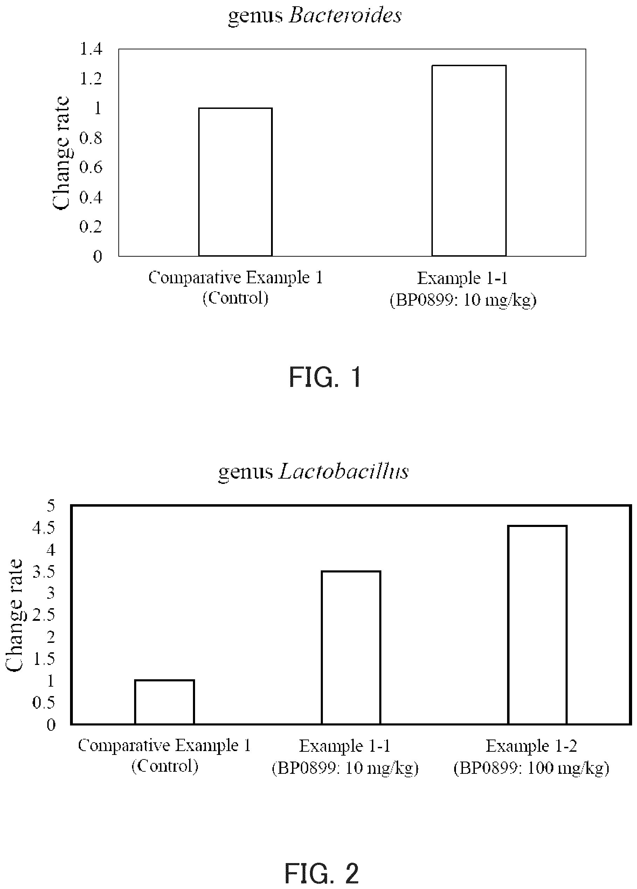

The intestinal microbiota distribution ratio (%) on day 3 was measured as follows. Feces of all of 7 mice in each group on day 3 were collected, and the collected feces were subjected to T-RFLP analysis with the number of specimen of n = 1. In the same manner, the intestinal microbiota distribution ratio (%) on day 7 was measured by collecting feces of all of 7 mice in each group on day 7 and subjecting the collected feces to T-RFLP analysis with the number of specimen of n = 1. Then, each change rate of the intestinal mictobiota distribution ratios in the mice was calculated as follows. The results of these are shown in FIGs. 1 to 6.

That is, as to the change in distribution ratio of genus *Bacteroides,* the value obtained by dividing the distribution ratio (%) of genus *Bacteroides* on day 7 by the distribution ratio (%) of genus *Bacteroides* on day 3 was calculated. Then, the relative value (change rate) in each of Examples 1-1 and 1-2 was calculated assuming that the value in Comparative Example 1 was 1. The change ratios of the distribution ratios of intestinal bacteria other than genus *Bacteroides* were also calculated in the same manner as for genus *Bacteroides.*

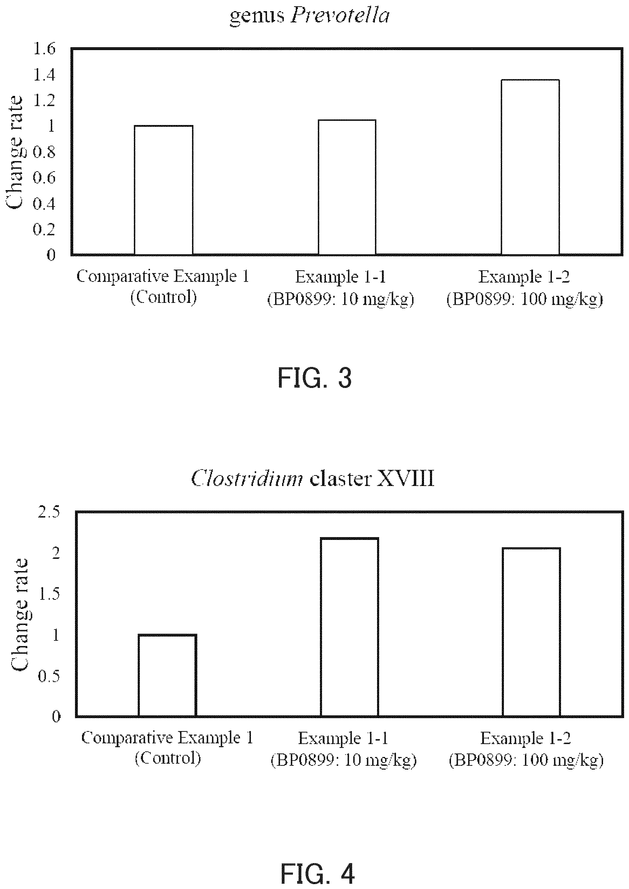

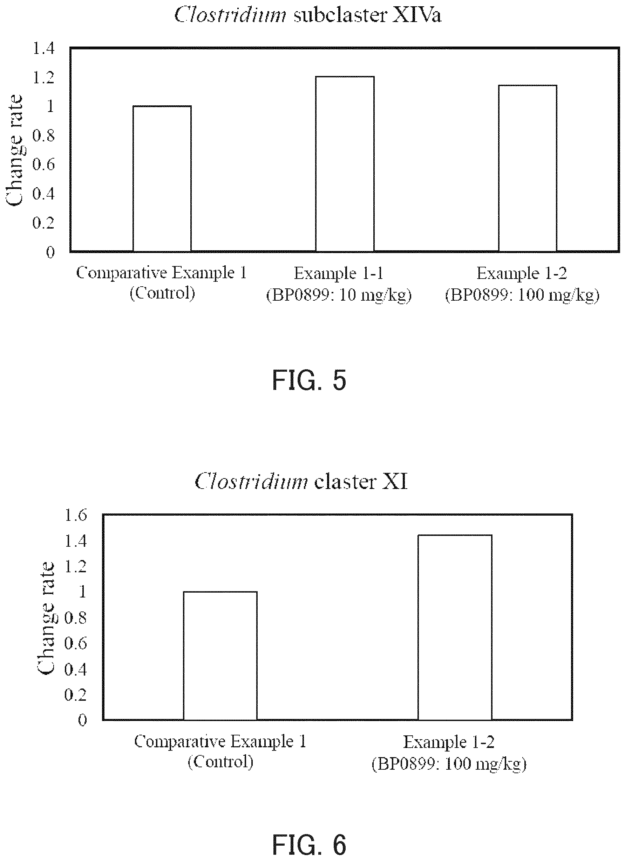

FIGs. 1 to 6 are graphs each showing the change in distribution ratio of intestinal bacteria in mice of Examples 1-1 and 1-2 and Comparative Example 1 by the kind of intestinal bacteria. FIG. 1 shows the results for genus *Bacteroides,* FIG. 2 shows the results for genus *Lactobacillus,* FIG. 3 shows the results for genus *Prevotella*, FIG. 4 shows the results for *Clostridium* claster XVIII, FIG. 5 shows the results for *Clostridium* subclaster XIVa, and FIG. 6 shows the results for *Clostridium* claster XI. In FIGs. 1 to 6, the vertical axis indicates the change rate.

In FIG. 1, the change rate of Example 1-1 was larger than 1, which showed that the distribution ratio of genus *Bacteroides* in Example 1-1 was larger than that of Comparative Example 1. In FIG. 2, the change rate of Example 1-1 was greatly larger than 1, which showed that the distribution ratio of genus *Lactobacillus* is significantly larger than that of Comparative Example 1. Moreover, the change rate of Example 1-2 was even larger than that of Example 1-1. This demonstrates the increase in distribution ratio of genus *Lactobacillus* in proportion to the amount of administering the BP0899 strain. In FIG. 3, the change rate of Example 1-1 was larger than 1, which showed that the distribution ratio of genus *Prevotella* was larger than that of Comparative Example 1. Moreover, the change rate of Example 1-2 was even larger than that of Example 1-1. This demonstrates the increase in distribution ratio of genus *Prevotella* in proportion to the amount of administering the BP0899 strain. In FIG. 4, the change rates of Examples 1-1 and 1-2 were larger than 1, which showed that the distribution ratios of *Clostridium* claster XVIII were larger than that of Comparative Example 1. In FIG. 5, the change rates of Examples 1-1 and 1-2 were larger than 1, which showed that the distribution ratios of *Clostridium* subclaster XIVa were larger than that of Comparative Example 1. In FIG. 6, the change rate of Example 1-2 was larger than 1, which showed that the distribution ratio of *Clostridium* claster XI was larger than that of Comparative Example 1. In the case where the distribution ratio of target intestinal bacteria on day 7 is higher than that on day 3 in Comparative Example 1, the change rates which are larger than 1 in Examples 1-1 and 1-2 mean that the extent of the increase is larger in Examples 1-1 and 1-2 than that in Comparative Example 1. In the case where the distribution ratio of target intestinal bacteria on day 7 is lower than that on day 3 in Comparative Example 1, the change rates of Examples 1-1 and 1-2 larger than 1 means that the extent of the decrease is smaller in Examples 1-1 and 1-2 than that in Comparative Example 1 (i.e., the decrease in target intestinal bacteria is inhibited in Examples 1-1 and 1-2), or the distribution ratio of target intestinal bacteria, which was reduced in Comparative Example 1, was increased in each of Examples 1-1 and 1-2.

### (3) Analysis 2 of feces (Comparison between distribution ratios of intestinal bacteria (%) on day 3 and das 8 to day 15)

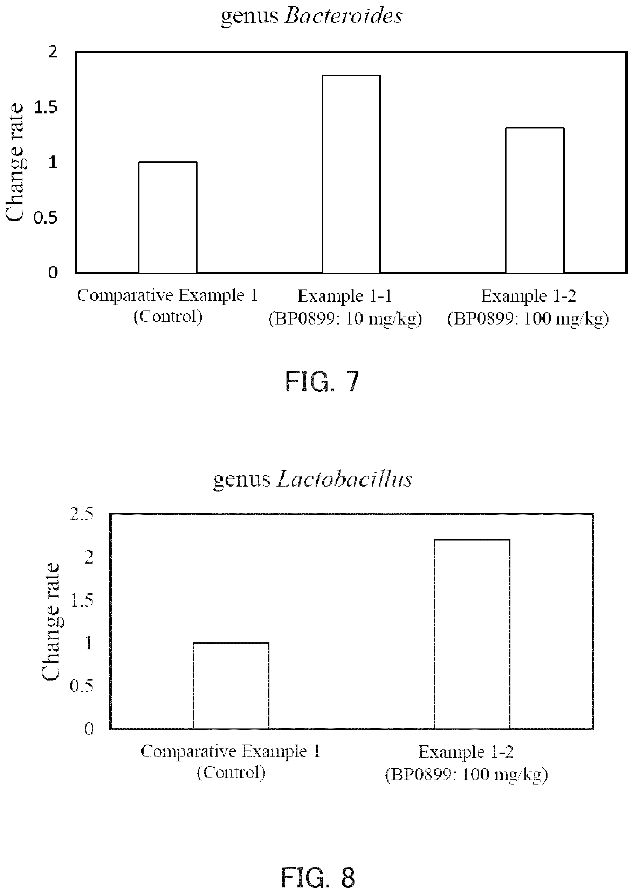

The distribution ratio (%) of intestinal bacteria on day 8 to day 15 was measured as follows. That is, among 7 mice in each group, three groups each including two mice were made. Then, feces of each two mice were collected from day 8 to day 15 (i.e., feces of each two mice were collected for 8 days), and the collected feces were subjected to T-RFLP analysis with the number of specimen of n = 3. Then, in the same manner as in the item (2) above, the value obtained by dividing the intestinal microbiota distribution ratio (%) on day 8 to day 15 by the intestinal microbiota distribution ratio (%) on day 3 was calculated, and the relative value (change rate) in each of Examples 1-1 and 1-2 was calculated assuming that the value in Comparative Example is 1. The results of these are shown in FIGS. 7 to 12.

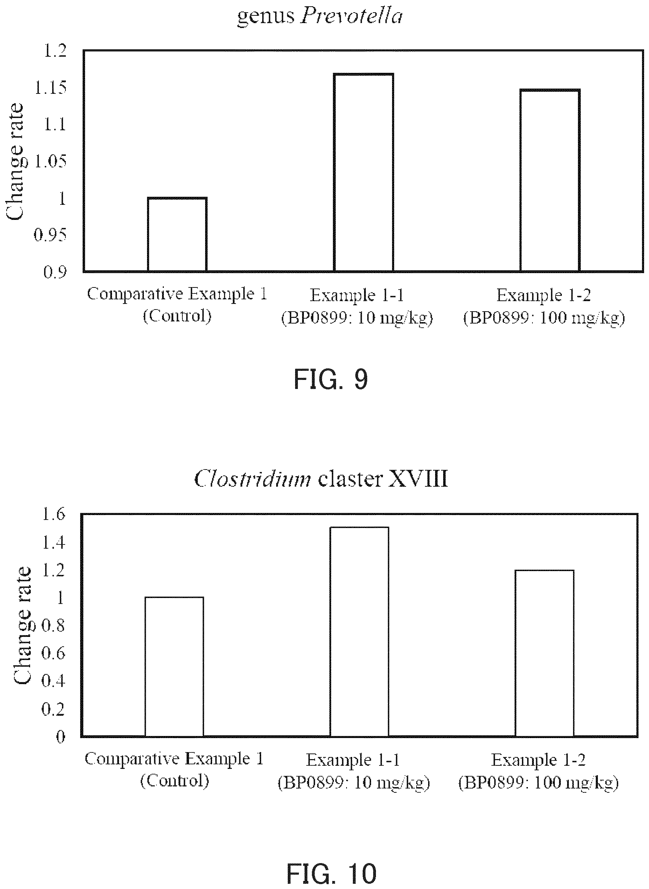

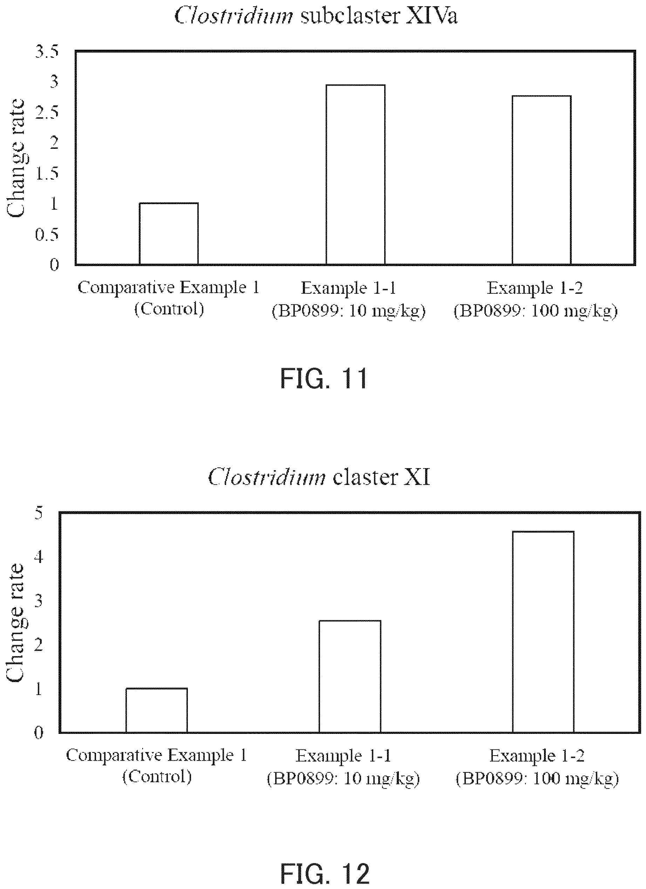

FIGs. 7 to 12 are graphs each showing the change in distribution ratio of intestinal bacteria in mice of Examples 1-1 and 1-2 and Comparative Example 1 by the kind of intestinal bacteria. FIG. 7 shows the results for genus *Bacteroides,* FIG. 8 shows the results for genus *Lactobacillus,* FIG. 9 shows the results for genus *Prevotella*, FIG. 10 shows the results for *Clostridium* claster XVIII, FIG. 11 shows the results for *Clostridium* subclaster XIVa, and FIG. 12 shows the results for *Clostridium* claster XI. In FIGs. 7 to 12, the vertical axis indicates the change rate.

In FIG. 7, the change rates of Examples 1-1 and 1-2 were larger than 1, which showed that the distribution ratio of genus *Bacteroides* was larger than that of Comparative Example 1. In FIG. 8, the change rate of Example 1-2 was larger than 1, which showed that the distribution ratio of genus *Lactobacillus* was larger than that of Comparative Example 1. In FIG. 9, the change rates of Examples 1-1 and 1-2 were larger than 1, which showed that the distribution ratio of genus *Prevotella* was larger than that of Comparative Example 1. In FIG. 10, the change rates of Examples 1-1 and 1-2 were larger than 1, which showed that the distribution ratio of *Clostridium* claster XVIII was larger than that of Comparative Example 1. In FIG. 11, the change rates of Examples 1-1 and 1-2 were greatly larger than 1, which showed that that distribution ratio of *Clostridium* subclaster XIVa was extremely larger than that of Comparative Example 1. In FIG. 12, the change rate of Example 1-1 was greatly larger than 1, which showed that the distribution ratio of *Clostridium* claster XI was extremely larger than that of Comparative Example 1. Moreover, the change rate of Example 1-2 was even larger than that of Example 1-1. This demonstrates the increase in distribution ratio of *Clostridium* claster XI in proportion to the amount of administering the BP0899 strain.

While the present invention is described above with reference to embodiments and examples, the present invention is not limited thereto. It will be understood by those of ordinary skill in the art that various changes in form and details may be made therein without departing from the spirit and scope of the present invention.

The present application claims priority to Japanese Patent Application No. 2016-088068, filed on April 26, 2016, and the entire content of the patent is hereby incorporated by reference.

### Industrial Applicability

As described above, the present invention can improve, for example, obesity and multiple sclerosis by regulating the intestinal microbiota distribution ratio with at least one of a *Rhodobacter azotoformans* BP0899 strain (Accession No. NITE BP-644) or a culture thereof. The agent for regulating the intestinal microbiota distribution ratio of the present invention is highly safe and thus can be administered for a long period of time. The present invention thus can provide a useful, highly-safe agent, pharmaceutical, and food and drink, for regulating the intestinal microbiota distribution ratio and the application range thereof is not limited and is wide.

## Claims

1. An agent for regulating the intestinal microbiota distribution ratio, comprising at least one of a *Rhodobacter azotoformans* BP0899 strain (Accession No. NITE BP-644) or a culture thereof.

2. The agent according to claim 1, wherein
the at least one of a *Rhodobacter azotoformans* BP0899 strain (Accession No. NITE BP-644) or a culture thereof comprises the following mycological characteristics (1) to (30):
(1) cell morphology: a rod shape or an oval shape;
(2) polymorphism: none;
(3) cell size: 0.8 µm × 1.0 µm;
(4) the presence or absence of motility: present;
(5) the presence or absence of spore: absent;
(6) luster in nutrient agar culture: positive;
(7) pigment production in nutrient agar culture: positive;
(8) the presence or absence of surface growth in nutrient broth culture: absent;
(9) the presence or absence of medium turbidity in nutrient broth culture: present;
(10) liquefaction of gelatin in gelatin stab culture: negative;
(11) coagulation in litmus-milk culture: negative;
(12) liquefaction in litmus-milk culture: negative;
(13) Gram staining properties: negative;
(14) reduction of nitrate: negative;
(15) denitrification: positive/negative;
(16) MR test: negative;
(17) production of indole: negative;
(18) production of hydrogen sulfide: negative;
(19) hydrolysis of starch: negative;
(20) utilization of citric acid (Christensen): negative;
(21) utilization of inorganic nitrogen source (ammonium salt): positive;
(22) production of catalase: positive;
(23) production of oxidase: positive;
(24) anaerobic growth: positive;
(25) O-F test (oxidation/fermentation): negative/negative;
(26) β-galactosidase activity: negative;
(27) arginine dihydrolase activity: negative;
(28) lysine decarboxylase activity: negative;
(29) tryptophan deaminase activity: negative; and
(30) gelatinase activity: negative.

3. The agent according to claim 1 or 2, wherein
a base sequence of the *Rhodobacter azotoformans* BP0899 strain (Accession No. NITE BP-644) is represented by SEQ ID NO: 1.

4. The agent according to any one of claims 1 to 3, wherein
the agent increases a distribution ratio or inhibits a reduction in the distribution ratio of at least one intestinal bacteria selected from the group consisting of genus *Bacteroides,* genus *Lactobacillus,* genus *Prevotella*, *Clostridium* claster XVIII, *Clostridium* subclaster XIVa, and *Clostridium* claster XI.

5. A pharmaceutical for regulating the intestinal microbiota distribution ratio, comprising the agent according to any one of claims 1 to 4.

6. Food or drink with the intestinal microbiota distribution ratio regulatory function, comprising the agent according to any one of claims 1 to 4.

7. A method for regulating the intestinal microbiota distribution ratio, comprising the step of administering an agent for regulating the intestinal microbiota distribution ratio, the agent comprising at least one of a *Rhodobacter azotoformans* BP0899 strain (Accession No. NITE BP-644) or a culture thereof.
